# EUROPÄISCHE PATENTSCHRIFT

(11) **EP 2 042 118 B1**
(45) Veröffentlichungstag und Bekanntmachung des Hinweises auf die Patenterteilung: **01.02.2012**
(21) Anmeldenummer: 08164841.2
(22) Anmeldetag: 23.09.2008
(51) Int. Cl.: A61B 18/14

(54) **Bipolares medizinisches Instrument**
Bipolar medical instrument
Instrument médical bipolaire

(30) Priorität: 25.09.2007 DE 102007047243
(43) Veröffentlichungstag der Anmeldung: 01.04.2009
(73) Patentinhaber: Karl Storz GmbH & Co. KG, 78532 Tuttlingen (DE)
(72) Erfinder: Bacher, Uwe, 78532 Tuttlingen (DE); Blocher, Martin, 78532 Tuttlingen (DE); Weinmann, Daniel, 78606 Seitingen- Oberflacht (DE)
(74) Vertreter: Witte, Weller & Partner

(56) Entgegenhaltungen:
- WO-A-94/08524
- DE-T2- 60 307 465
- US-A- 5 697 949

## Beschreibung

Die Erfindung betrifft ein bipolares medizinisches Instrument, mit einem Schaft, mit zwei Maulteilen, die relativ zueinander beweglich an einem distalen Ende des Schaftes angeordnet sind, wobei Maulteile jeweils eine mit Hochfrequenzstrom beaufschlagbare Elektrode unterschiedlicher Polarität bilden, wobei jedem Maulteil eine getrennte Stromzuleitung zugeordnet ist, von denen eine über den Schaft und die andere durch ein in dem Schaft angeordnetes, axial bewegliches Kraftübertragungselement, das kraftsschlüssig mit zumindest einem Maulteil verbunden ist, gebildet wird, wobei die Maulteile elektrisch voneinander isoliert sind, und wobei ein distaler Endbereich des Kraftübertragungselementes von einem kappenartigen oder hülsenartigen isolierenden Element umschlossen ist, das aus einem elektrisch isolierenden Material ausgebildet und an dem Kraftübertragungselement angebracht ist, und wobei das isolierende Element gelenkig mit zumindest einem Maulteil verbunden ist, wobei ein elektrisch leitendes erstes Kontaktelement vorhanden ist, mittels dessen der Stromfluss von dem Rohrschaft auf das eine Maulteil bewerkstelligbar ist.

Ein derartiges bipolares medizinisches Instrument ist aus der DE 601 13 269 T2 bekannt.

Bipolare medizinische Instrumente werden dazu verwendet, Gewebe im menschlichen oder tierischen Körper unter Einwirkung von bipolarem Hochfrequenzstrom thermisch zu schneiden, zu koagulieren oder zu schneiden und zu koagulieren.

Üblicherweise weisen derartige bipolare Instrumente einen Rohrschaft auf, an dessen distalem Ende gelenkig miteinander verbundene Maulteile angeordnet sind, die relativ zueinander beweglich sind. In dem Rohrschaft ist ein axial bewegliches Kraftübertragungselement angeordnet, das proximalseitig mit einer Handhabe und distalseitig mit zumindest einem Maulteil, nämlich dem beweglichen Maulteil, kraftschlüssig verbunden ist. Durch Betätigen der Handhabe können die Maulteile geöffnet oder geschlossen werden. Die Maulteile bilden jeweils eine mit Hochfrequenzstrom beauschlagbare Elektrode unterschiedlicher Polarität aus. Jedem Maulteil ist eine getrennte Stromzuleitung zugeordnet, die mit jeweils einem Pol der Hochfrequenzspannungsquelle verbunden ist. In den meisten Fällen dient das Kraftübertragungselement als elektrisch leitende Verbindung zu dem beweglichem Maulteil, wohingegen der Rohrschaft als elektrisch leitende Verbindung zum anderen Maulteil, sei es ein bewegliches oder ein unbewegliches, dient, siehe z.B. US-A-5 697 949.

Bei solchen bipolaren Instrumenten muss nicht nur eine ausreichende elektrische Isolierung zwischen den beiden Stromzuleitungen, sondern insbesondere zwischen den beiden Maulteilen im Bereich deren Anlenkstellen vorhanden sein, um beim Einsatz Kurzschlüsse über Kriechströme zu vermeiden.

Bei der eingangs genannten DE 601 13 296 T2 ist auf dem distalen Endbereich des Kraftübertragungselements ein hülsenartiges isolierendes Element aufgeschoben. Von diesem springt nach distal ein Abschnitt vor, an den die beiden Maulteile gegenüberliegend und gelenkig angebracht sind.

Um dasjenige Maulteil, das über das Kraftübertragungselement mit der Stromquelle versorgt wird, elektrisch zu verbinden, ist ein Kontaktelement vorgesehen, das etwa halbschalenförmig um das isolierende Element liegt und einerseits elektrisch leitend mit dem Kraftübertragungselement und andererseits mit der Bewegungsmechanik des entsprechenden Maulteils verbunden ist. Ein äußerer an die Außenseite des halbschalenförmigen Abschnittes des Kontaktelements angelegter Isolator dient zur Isolierung gegenüber dem Schaft.

Um dasjenige Maulteil, das über den Schaft mit der Stromquelle elektrisch versorgt wird, zu verbinden, ist ein weiteres elektrisch leitendes Kontaktelement vorhanden, das distalseitig mit der Außenseite des entsprechenden Maulteiles verbunden ist. Dabei erfolgt die Verbindung ebenfalls über die Bewegungsmechanik dieses Maulteils. Auch dieses weitere Kontaktelement weist einen halbschalenförmigen Abschnitt auf, der etwa dem halbschalenförmigen Abschnitt des anderen Kontaktelements gegenüberliegt.

Zwischen das weitere Kontaktelement ist ein weiterer etwa halbschalenförmiger Isolator gelegt, der für die Isolierung dieses ersten Kontaktelements gegenüber dem diametral gegenüberliegenden Strompfad sorgen soll, der über das Kraftübertragungselement das zweite isolierende Element das entsprechende Maulteil läuft.

Problematisch an dieser Konstruktion der Stromübertragung vom Kraftübertragungselement zum entsprechenden Maulteil bzw. vom Schaft zum entsprechenden Maulteil ist, dass die entsprechenden Kontaktelemente etwa halbschalenförmig ausgebildet sind und sich nahe gegenüberliegen. Diese Kontaktelemente tragen auch zugleich die Schwenkzapfen, um die die Maulteile verschwenkt werden. Dazu müssen sie entsprechend massiv und stabil ausgebildet werden, so dass sie auch eine gewisse räumliche Ausdehnung erfordern. Das bedeutet, dass konstruktionsbedingt der räumliche Abstand zwischen den beiden Kontaktelementen, die für die Kontaktierung zwischen Kraftübertragungselement und dem einen Maulteil bzw. Schaft und dem anderen Maulteil sorgen, relativ gering ist.

Im Einsatz treten diese medizinischen Instrumente insbesondere im Bereich der Maulteile mit elektrisch leitenden Flüssigkeiten, insbesondere mit Körperflüssigkeiten, in Kontakt, die die Ausbildung von Kriechströmen fördern, so dass Kurzschlüsse nicht ausgeschlossen werden können. Üblicherweise wird an die Maulteile eine Hochfrequenzspannung in der Größenordnung von 2,5 kV gelegt, das bedeutet, dass bei einer kleinen Bauweise und entsprechend geringen Abständen ein Spannungsüberschlag erfolgen kann oder sich ein Kriechstrom über die isolierenden Elemente hinweg zum nächsten leitenden Bauteil ausbildet, was zu einem Kurzschluss führt.

Durch die relativ bulkige und relativ lange Ausbildung der Kontaktelemente bei der eingangs genannten DE 601 13 297 T2 sind solche Störfaktoren konstruktionsbedingt nicht vollständig auszuschließen.

In der DE 603 07 465 T2 ist eine weitere sehr ähnlich ausgebildete bipolare elektrochirurgische Zange mit nichtleitenden Abstandhaltern gemäß Oberbegriff des Anspruchs 1 beschrieben.

Aus der DE 196 08 716 C1 ist bekannt, das Kraftübertragungselement über jeweils ein Kniehebelelement mit einem Maulteil von zwei Maulteilen gelenkig und elektrisch leitend zu verbinden. Um zu vermeiden, dass ein Stromfluss an dem Kraftübertragungselement über die elektrisch leitenden Kniehebelelemente an beide Mauleile erfolgt, ist ein zweites Kniehebelelement des Maulteiles, das mit dem Rohrschaft leitend verbunden ist, aus einem elektrisch isolierenden Material ausgebildet.

Aus der DE 694 32 937 T2 bzw. der WO 94/08524 A ist ein chirurgisches Instrument bekannt, bei dem die beiden Maulteile über separate elektrische Leiter mit der Stromquelle verbunden sind.

Auch bei dem aus der DE 198 58 512 C1 bekannten bipolaren medizinischen Instrument werden die Maulteile über separate Stromzuleitungen versorgt. Isolatoren sorgen dafür, dass die Maulteile voneinander elektrisch isoliert sind.

Aus der DE 199 40 689 A1 ist ein bipolares medizinisches Instrument bekannt, das ein feststehendes und ein bewegliches Maulteil aufweist. Das feststehende Maulteil weist einen einstückigen Grundkörper auf, der aus einem elektrisch isolierenden Material ausgebildet ist. Auf diesen Grundkörper ist ein Maulteileinsatz aufgebracht, der elektrisch leitend ausgebildet ist. Dieser ist elektrisch leitend über ein Drahtelement mit dem Schaft verbunden. Das zweite elektrisch leitende Element ist mit einer separaten Stromversorgung versehen.

Aus der DE 10 2004 015 667 B3 ist ein bipolares Doppelgelenkinstrument bekannt, das eine Kniehebelsteuerung zur Bewegung der beiden Maulteile aufweist. Die beiden Maulteile bestehen aus metallischem Material und werden durch ein Zugstangenisolierteil sowie ein weiteres Isolierformteil elektrisch voneinander getrennt.

Die US 6,063,086 beschäftigt sich mit der Isolierung zweier bewegbarer Arme an einem bipolaren endoskopischen Instrument. Die Arme sind über elektrische Leiter mit einer Energiequelle verbunden, wobei der Schaft, um den sich die Arme drehen, zwei voneinander elektrisch isolierte, für sich gesehen leitfähige Abschnitte zur Stromübertragung aufweist.

Aus der DE 201 18 302 U1 ist ein chirurgisches endoskopisches HF-Instrument bekannt, das distalseitig eine Gabel aufweist, in der zwei Maulteile aufgenommen sind. Dabei ist die Gabel an ihrer äußeren elektrisch leitenden Fläche mit einer Isolierbeschichtung versehen.

Es ist Aufgabe der vorliegenden Erfindung, ein bipolarisches medizinisches Element der eingangs genannten Art derart weiterzuentwickeln, dass eine auf Dauer sichere Isolierung der Maulteile im Bereich des Gelenkes bewerkstelligt wird, und dass eine mechanisch stabile Kraftübertragung auf Dauer gewährleistet ist.

Erfindungsgemäß wird die Aufgabe dadurch gelöst, dass das erste Kontaktelement an dem isolierenden Element fixiert ist.

Diese Maßnahme hat den Vorteil, dass das erste Kontaktelement, das den Stromfluss von dem Rohrschaft an das zugehörige Maulteil überträgt, durch das isolierende Element von dem Kraftübertragungselement elektrisch isoliert ist. Dies trägt nicht nur zu einer sicheren elektrischen Trennung der beiden Strompfade bei, sondern das isolierende Element dient auch als mechanische Stütze oder Träger für diese Kontaktierung.

Die Fixierung sorgt für eine unverrückbare Positionierung und erlaubt es auch, dieses Kontaktelement sehr schlank auszubilden, gerade ausreichend um den Strom von dem Schaft zu dem entsprechenden Maulteil zu leiten. Die eigentliche Kraftübertragung erfolgt über das isolierende Element, d.h. die Hin- und Herbewegung des Kraftübertragungselementes wird in eine Hin- und Herbewegung des daran angebrachten isolierenden Elementes umgesetzt, an dem wiederum die Maulteile angelenkt sind.

Das erste Kontaktelement ist somit kein Bestandteil des Kraftübertragungspfades, ist aber an demjenigen Element, das die Kraftübertragung des Kraftübertragungselements weiterleitet, fixiert. Dadurch, dass dieses Element aus einem isolierenden Material hergestellt ist, ist gleichzeitig eine sichere Isolierung gegenüber dem Kraftübertragungselement, das den anderen Pol darstellt, geschaffen.

Dadurch, dass nun dieses erste Kontaktelement sehr schlank ausgebildet werden kann, weil es nicht an der Kraftübertragung teilnimmt, können die Kriechstromstrecken, also der Abstand zu dem anderen Kontaktelement, relativ groß ausgebildet werden, so dass die Ausbildung von Kriechströmen, beispielsweise über leitende Körperflüssigkeiten, dramatisch reduziert werden kann. Da relativ wenig und kleine Teile benötigt werden erlaubt dies auch eine äußerst schlanke Konstruktion des bipolaren medizinischen Instrumentes.

Durch die gelenkige Verbindung des isolierenden Elementes mit einem Maulteil kann die Kraft, die vom Kraftübertragungselement ausgeübt wird, über das isolierende Element auf das mit diesem verbundene Maulteil übertragen werden. Eine kappenartige oder hülsenartige Ausbildung erlaubt eine großflächige Isolierung zwischen dem leitenden Kraftübertragungselement und dem von diesem bewegten Maulteil.

Zugleich ist es auch bei kleinster Bauweise möglich, die Pole vom leitenden Kraftübertragungselement und dem leitenden, gelenkig am isolierenden Element angebrachten gegenpoligen Maulteil sehr weit voneinander zu beabstanden, so dass Kriechströme dort weitgehend unterbunden werden können. Das isolierende Element kann auch bei an sich schlanker Bauweise des Instrumentes ausreichend lang und stabil ausgebildet werden, um die Doppelfunktion, nämlich die ausreichende Isolierung und auf Dauer mechanisch stabile Kraftübertragung zu erfüllen.

Somit stehen auch ausreichend Fixierpunkte zur Verfügung, um das erste Kontaktelement an diesem isolierenden Element anzubringen, so dass dies seine Funktion, nämlich eine Kontaktierung des Schaftes mit dem entsprechenden Maulteil, lagestabil und isolierend gegenüber dem anderen Pol durchführen kann.

In einer weiteren Ausgestaltung der Erfindung ist ein weiteres, einen Stromfluss von dem Kraftübertragungselement an das andere Maulteil schaffendes zweites Kontaktelement vorhanden, das in Bezug auf das erste Kontaktelement umfänglich versetzt angeordnet ist.

Diese Maßnahme hat den Vorteil, dass durch das weitere Kontaktelement der Stromfluss von dem Kraftübertragungselement an das zugehörige Maulteil geschaffen wird, und gleichzeitig die beiden Kontaktelemente einen möglichst großen Abstand voneinander aufweisen, um Spannungsüberschläge auszuschließen.

In einer weiteren Ausgestaltung der Erfindung ist dieses zweite Kontaktelement über einen Kontakt mit dem Kraftübertragungselement verbunden, wobei der Kontakt als zumindest ein durch einen Hülsenabschnitt des isolierenden Elements durchreichenden Stift ausgebildet ist. Bevorzugt können zwei Stifte vorhanden sein.

Diese Maßnahme hat den Vorteil, dass über die Stifte nicht nur eine mechanisch feste Anbindung und somit Lagefixierung dieses weiteren Kontaktelements sichergestellt ist, sondern gezielt über die zwei Stifte Durchbruchstellen im isolierenden Element geschaffen sind, um von dem in diesem aufgenommenen leitenden Kraftübertragungselement Strom über das weitere zweite Kontaktelement zu dem entsprechenden Maulteil zu leiten. Die Lage der Stifte kann dann so gewählt werden, dass diese Kontaktstelle möglichst weit von der Kontaktstelle entfernt ist, die den Stromfluss vom Rohrschaft zu dem anderen Maulteil bewerkstelligt, so dass auch hier wieder maximal lange Kriechstromstrecken geschaffen werden.

In einer weiteren Ausgestaltung der Erfindung ist zumindest ein Maulteil an der Seite, die dem anderen Maulteil zugewandt ist, zumindest im Bereich einer Anlenkung der beiden Maulteile untereinander mit einer Isolierung versehen.

Diese Maßnahme hat den Vorteil, dass die beiden Maulteile zumindest in dem Bereich, in dem sie jeweils angelenkt sind und sich vis-ä-vis stehen, großflächig durch diese Beschichtung voneinander isoliert sind. Die beiden Maulteile selbst können metallisch und somit mechanisch stabil ausgebildet sein, auch im Anlenkungsbereich, so dass die Kräfte beim Öffnen und Schließen durch mechanisch stabile Teile übertragen werden können.

Auch kann der Gelenkzapfen, über den die beiden Maulteile gelenkig verbunden sind, metallisch ausgebildet werden, er muss allerdings dann zumindest im Bereich der Lagerung von einer isolierenden Umhüllung umgeben sein, wie das an sich bekannt ist, um in diesem Bereich ebenfalls eine Isolierung zu gewährleisten.

In einer weiteren Ausgestaltung der Erfindung bestehen die elektrisch isolierenden Materialen aus einem Keramikwerkstoff, vorzugsweise einem Keramikwerkstoff mit hoher Härte und geringer Sprödigkeit.

Diese Maßnahme hat den Vorteil, dass die elektrisch isolierenden Elemente eine besondere hohe mechanische Beständigkeit besitzen, so dass die Doppelfunktion, also Kraftübertragung einerseits und Isolierung andererseits, auf lange Dauer gewährleistet ist.

In einer weiteren Ausgestaltung der Erfindung ist das isolierende Element gelenkig mit beiden Maulteilen verbunden.

Diese Maßnahme hat den Vorteil, dass das isolierende Element zur Kraftübertragung auf beide Maulteile herangezogen werden kann. Die gelenkige Verbindung kann dann aus metallischen Teilen bestehen, die ausreichend stabil sind, um die Kräfte weiter zu übertragen, beispielsweise über einen Kniehebelmechanismus. Dennoch ist eine ausreichende Isolierung in diesem Bereich sowohl in Richtung auf das leitende Kraftübertragungselement, als auch eine Isolierung zwischen den beiden gelenkigen Verbindungen untereinander gewährleistet. Dies zeigt besonders beeindruckend die Eignung des isolierenden Elements zur Erfüllung der zuvor genannten Doppelfunktion.

In einer weiteren Ausgestaltung der Erfindung weist das isolierende Element einen Hülsenabschnitt auf, der auf den distalen Endbereich des Kraftübertragungselements aufgebracht ist.

Diese Maßnahme hat den Vorteil, dass auch bei schlankester Bauweise eine entsprechend mechanisch stabile Verbindung zwischen dem Kraftübertragungselement und dem isolierenden Element hergestellt werden kann, somit auf Dauer die Kräfte übertragen werden können. Der Hülsenabschnitt stellt nun einen großflächigen Isolierbereich dar, der es erlaubt, die Kontaktstellen zum Beaufschlagen der beiden Maulteile mit unterschiedlicher Polarität möglichst weit voneinander zu beabstanden und somit maximal lange Kriechstromstrecken zu etablieren, wodurch Kurzschlüsse vermieden werden können. Wie bereits erwähnt, kann der Hülsenabschnitt auch als ein Beschichtungsabschnitt ausgebildet sein.

In einer weiteren Ausgestaltung der Erfindung weist das isolierende Element einen T-Abschnitt auf, an dem die gelenkige Verbindung mit dem bzw. den Maulteilen bewerkstelligbar ist.

Diese Maßnahme hat den Vorteil, dass der T-Abschnitt auch bei schlanker Bauweise mechanische Anlenkungspunkte für die gelenkige Verbindung mit den Maulteilen bzw. einem dazwischen geschalteten Gelenk, beispielsweise einem Kniehebelmechanismus, bietet. Wie erwähnt, kann eine ausreichend mechanisch stabile gelenkige Verbindung in diesem T-Bereich bewerkstelligt werden und zugleich wieder relativ große Abstände zwischen den an sich leitenden gelenkig angebrachten Bauelementen geschaffen werden, um in diesem Bereich Spannungsüberschläge zu vermeiden. Dies kann dadurch erfolgen, dass die Anlenkungspunkte jeweils an den äußeren gegenüberliegenden Enden des T-Abschnittes liegen.

Weitere Vorteile ergeben sich aus der nachfolgenden Beschreibung im Zusammenhang mit den beigefügten Zeichnungen.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese nachfolgend näher beschrieben. Es zeigen:
- Figur 1: eine Seitenansicht eines erfindungsgemäßen bipolaren medizinischen Instrumentes;
- Figur 2: eine Seitenansicht eines Teiles des distalen Endes des Instrumentes, wobei die mechanische Kraftübertragung zum Öffnen und Schließen der Maulteile dargestellt ist, die in geschlossenem Zustand gezeigt sind;
- Figur 3: eine der Figur 2 vergleichbare Darstellung mit geöffneten Maulteilen;
- Figur 4: einen Mittellängsschnitt des distalen Endbereiches des erfindungs- gemäßen Instrumentes von Figur 1;
- Figur 5: einen gegenüber der Darstellung von Figur 4 um 90° um die Mittelach- se verdrehten abschnittsweise etwas vergrößerten Längsschnitt des distalen Endbereiches des Instrumentes von Figur 1; und
- Figur 6: eine vergrößerte perspektivische Ansicht der beiden Maulteile.

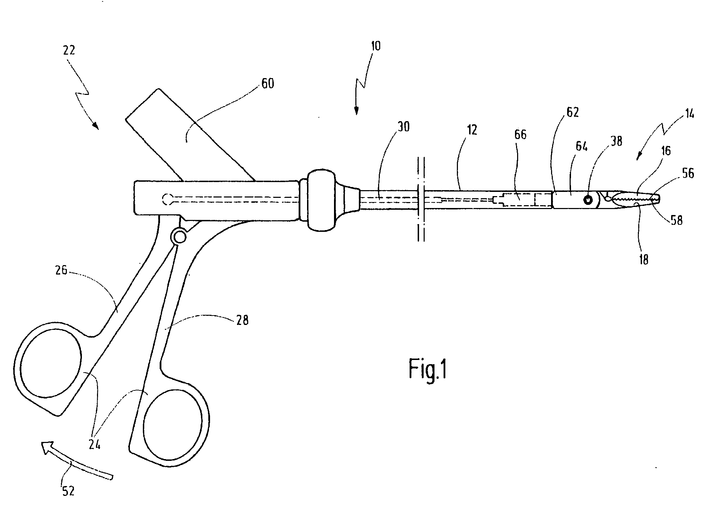

Ein in Figur 1 dargestelltes bipolares medizinisches Instrument ist in seiner Gesamtheit mit der Bezugsziffer 10 bezeichnet. Das Instrument 10 findet im Rahmen von minimal-invasiven chirurgischen Eingriffen zur Behandlung von Gewebe im menschlichen oder tierischen Körper zur Präparation mittels Hochfrequenzstrom Einsatz.

Das in Figur 1 dargestellte Ausführungsbeispiel des Instrumentes 10 ist ein Fassinstrument bzw. eine Fasszange.

Das Instrument 10 weist einen langerstreckten Rohrschaft 12 auf. Der Rohrschaft 12 ist aus einem elektrisch leitenden Material, bevorzugt aus Metall, ausgebildet.

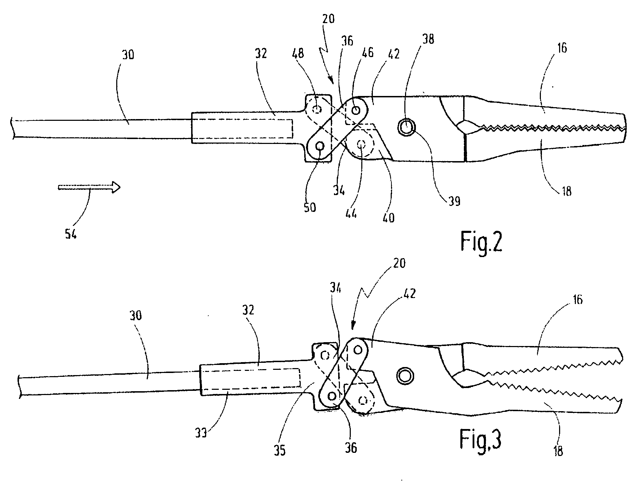

An einem distalen Ende 14 des Rohrschaftes 12 sind zwei Maulteile 16, 18 angeordnet. Die Maulteile 16, 18 sind über einen Anlenkzapfen 38 miteinander verbunden und relativ zueinander beweglich, wie das später noch im Zusammenhang mit Figuren 2 und 3 beschrieben wird.

Am distalen Endbereich des Instruments 10 ist ein Gehäuse 62 vorhanden. Ein distaler gabelkopfartiger Bereich 64 des Gehäuses 62 ist aus einem elektrisch isolierenden Material ausgebildet, wohingegen ein proximalseitiger Bereich 66 des Gehäuses 62 aus einem elektrisch leitenden Material ausgebildet ist. Dieser proximalseitige Bereich 66 dient zur elektrisch leitenden Verbindung mit dem Rohrschaft 12 und ist dazu in diesen eingesteckt. Die Maulteile 16 und 18 sind über den Anlenkzapfen 38 mit dem gabelkopfartigen Bereich 64 verbunden.

Das Instrument 10 weist ferner an seinem proximalen Ende eine Handhabe 24 auf. Die Handhabe 24 besteht in diesem Ausführungsbeispiel aus zwei Griffteilen 26 und 28, wobei das Griffteil 26 beweglich und das Griffteil 28 unbeweglich ausgebildet ist.

Zwischen dem beweglichen Griffteil 26 und den Maulteilen 16 und 18 erstreckt sich in dem Rohrschaft 12 ein Kraftübertragungselement 30, das in diesem Ausführungsbeispiel in Form einer Schub- und Zugstange ausgebildet ist. Das Kraftübertragungselement 30 ist im Rohrschaft 12 axial beweglich ausgebildet.

Das Kraftübertragungselement 30, das aus einem elektrisch leitenden Material ausgebildet ist, ist distalseitig von einem isolierenden Element 32 kappenartig umschlossen, wie das insbesondere aus der Darstellung in Figur 2 und 3 ersichtlich ist.

Das isolierende Element 32 ist aus einem elektrisch isolierenden, insbesondere einem keramischen oder einem keramisch umschlossenen Material ausgebildet und ist gelenkig mit den beiden Maulteilen 16, 18 verbunden. Ein Gelenk 20 ist als eine Kniehebelanordnung ausgebildet und dient zur Steuerung und Bewegung der Maulteile 16 und 18 durch das Kraftübertragungselement 30.

Die beiden Maulteile 16 und 18 sind als um den Gelenkzapfen 38 schwenkbare Hebel ausgebildet. Ein proximaler Abschnitt 40, 42 jedes Maulteils 16, 18 ist mittels jeweils einem Niet 44, 46 mit jeweils einer Gelenklasche 34, 36 gelenkig verbunden, wie es aus der Darstellung in Figur 2 und 3 hervorgeht. Die Gelenklaschen 34, 36 sind an ihrem anderen Ende mittels jeweils einem Niet 48, 50 mit einem distalen Ende des isolierenden Elements 32 gelenkig verbunden. Der Zapfen 38, die Gelenklaschen 34, 36 und die Niete 44, 46, 48 und 50 sind aus metallisch leitendem Material, vorzugsweise aus Metall, ausgebildet.

In der Ansicht von Figur 2 und 3 ist die Gelenklasche 36 am "oberen" Ende des isolierenden Elements angelenkt, wohingegen die Gelenklasche 34 diametral gegenüberliegend am "unteren" Ende angelenkt ist.

Das isolierende Element 32 ist als T-Stück ausgebildet. Das Niet 50, das über die "obere" Gelenklasche 36 am T-Stück angenietet ist, liegt am gegenüberliegenden Ende des Querbalkens des T-Stücks, an dem das Niet 48 liegt, über das die "untere" Gelenklasche 34 annietet.

Die Gelenklaschen 34 und 36 kreuzen sich, sind jedoch voneinander beabstandet. Die beabstandeten Anlenkungspunkte der Nieten 48 und 50 sorgen für eine elektrische Isolierung dieser Bauelemente gegeneinander. Dies erlaubt es, die Gelenklaschen 34 und 36 aus Metall, somit mechanisch stabil, auszubilden, um die Kräfte zum Öffnen und Schließen der Maulteile 16 und 18 zu übertragen. Die "obere" Gelenklasche 36 ist mit dem "oberen" Maulteil 18 verbunden, wohingegen die "untere" Gelenklasche 34 mit dem "unteren" Maulteil 16 verbunden ist.

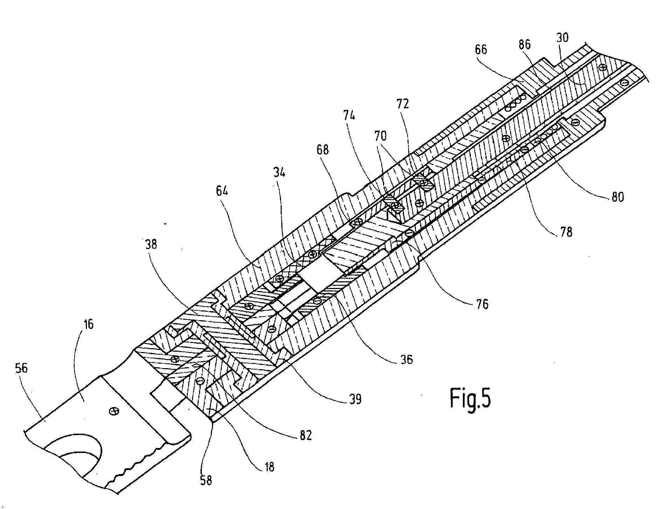

Der Anlenkzapfen 38, der die beiden Maulteile 16 und 18 gelenkig bzw. um diesen schwenkbar miteinander verbindet, ist von einer zweiteiligen Steckhülse 39 aus isolierendem Material umgeben (siehe Figur 5).

Dadurch wird eine elektrische Isolierung zwischen den Maulteilen 16 und 18 um deren Anlenkzapfen 38 geschaffen. Der Anlenkzapfen 38 selbst ist aus einem metallischen Material hergestellt.

Die besondere Ausgestaltung des isolierenden Elements 32 erlaubt es, die unterschiedlich gepolte Stromversorgung der Maulteile 16 und 18 möglicht weit voneinander zu beabstanden.

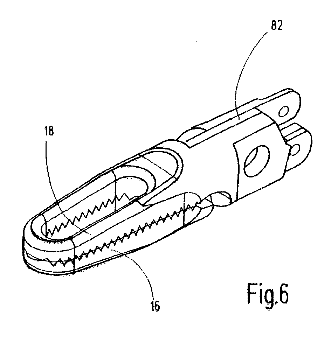

Zur zusätzlichen Isolierung ist zumindest eine der sich im Anlenkungsbereich gegenüberliegenden Flächen der Maulteile 16 und 18 mit einer isolierenden Beschichtung versehen. Im dargestellten Ausführungsbeispiel ist das Maulteil 18 mit einer isolierenden Beschichtung 82 (siehe Figur 6) versehen.

Um die Maulteile 16, 18 zu öffnen, wird das bewegliche Griffteil 26 der Handhabe 24 in die Richtung, die durch einen Pfeil 52 in Figur 1 angezeigt ist, verschwenkt. Das bewegliche Griffteil 26 ist mit einem proximalen Ende des Kraftübertragungselements 30 kraftschlüssig verbunden, beispielsweise durch ein Kugelpfannengelenk, wie das an sich bekannt ist. Durch eine derartige kraftschlüssige Verbindung wird eine Schwenkverbindung des Griffteiles 26 in eine lineare Verschiebebewegung des Kraftübertragungselements 30 und somit auch des isolierenden Elements 32 umgesetzt (siehe Pfeil 54 in Figur 2). Diese Verschiebebewegung bewirkt eine Bewegung der Gelenklaschen 34 und 36, die jeweils mit einem der Maulteile 16 bzw. 18 gelenkig verbunden sind. Die Bewegung der Gelenklaschen 34 und 36 bewirkt ein Verschwenken der Maulteile 16 und 18 um deren Anlenkzapfen 38, so dass die Maulteile 16, 18 in einen gespreizten Zustand versetzt werden. Diese Situation ist in Figur 3 dargestellt.

Eine entgegengesetzte Verschiebebewegung bewirkt dann das Schließen der Maulteile 16 und 18.

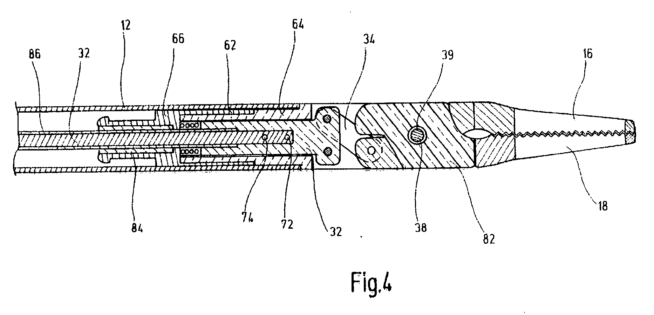

In Zusammenhang mit den Figuren 4 und 5 soll die Stromversorgung der beiden Maulteile 16 und18 näher beschrieben werden.

Die Maulteile 16 und 18, die in diesem Ausführungsbeispiel vollständig aus einem elektrisch leitenden Material, vorzugsweise aus Metall, hergestellt sind, bilden zwei mit Strom beaufschlagbare Elektroden 56, 58 unterschiedlicher Polarität aus. Eine Elektrode 56 ist im Betrieb mit einem Pol einer Hochfrequenzspannungsquelle verbunden, wohingegen die Elektrode 58 mit dem anderen Pol der Hochfrequenzspannungsquelle verbunden ist.

Um die Maulteile 16 und 18 mit Strom zu versorgen, weist das Instrument 10 an dem proximalen Ende 22 einen schrägstehenden Steckeranschluss 60 auf, über den das Instrument 10 an eine hier nicht dargestellte externe Hochfrequenzspannungsquelle angeschlossen werden kann.

Das Kraftübertragungselement 30 dient als elektrisch leitende Verbindung zu dem ersten Maulteil 16, wohingegen der Rohrschaft 12 als elektrisch leitende Verbindung für das zweite Maulteil 18 dient, was nachfolgend näher beschrieben wird.

An dem isolierenden Element 32 ist an einer Außenseite ein elektrisch leitendes streifenförmiges zweites Kontaktelement 68 fixiert, das über zwei Kontaktstifte 72, 74, die quer durch einen Hülsenabschnitt 33 des isolierenden Elements 32 ragen, mit dem Kraftübertragungselement 30 leitend verbunden ist.

Distalseitig ist das Kontaktelement 68 mit der Gelenklasche 34 des Maulteils 16 leitend verbunden, wie das insbesondere aus der Darstellung von Figur 5 hervorgeht.

Somit erfolgt ein Stromfluss zwischen dem Kraftübertragungselement 30 und dem ersten Maulteil 16 über das zweite Kontaktelement 68 und die Gelenklasche 34. Dieser Stromfluss ist in Figur 5 zum besseren Verständnis mit Pluszeichen angedeutet.

Wie bereits zuvor erwähnt, dient der äußere Rohrschaft 12 als elektrisch leitende Verbindung für das zweite Maulteil 18. Der Stromfluss wird von dem Rohrschaft 12 an den proximalseitigen Bereich 66 des Gehäuses 62 geleitet, der vom Rohrschaft 12 umschlossen ist und mit dem Rohrschaft 12 leitend verbunden ist (siehe Figur 1).

Dieses weitere erste Kontaktelement 76 ist in Bezug auf das zuvor beschriebene zweite Kontaktelement 68 umfänglich versetzt angeordnet, vorzugsweise um 180° angeordnet, liegt also diametral gegenüber und ist von diesem durch das isolierende Element 32 elektrisch isoliert.

Das weitere erste Kontaktelement 76 ist über einen Kontakt 78 mit dem proximalseitigen Bereich 66 des Gehäuses 62 leitend verbunden. Das erste Kontaktelement 76 ist am isolierenden Element 32 fixiert, z.B. durch eine Verklebung oder durch mechanische Ankerstellen, und folgt somit dessen linearen Bewegungen. Der Kontakt 78 ist als federbelasteter Kontakt 80 ausgebildet, um eine effektive Kontaktierung zwischen dem Gehäuse 62 und dem Rohrschaft 12 bei solchen Bewegungen zu gewährleisten. Von dem Kontaktelement 76 wird der Stromfluss an die Gelenklasche 36 und dann an das zweite Maulteil 18 übertragen. Das Kontaktelement 76 kann als schmaler, länglicher Metallstreifen ausgebildet sein. Er kann auch drahtförmig sein.

Somit erfolgt der Stromfluss zwischen dem Rohrschaft 12 und dem Maulteil 18 über den proximalseitigen Bereich 66 des Gehäuses 62, den federbelasteten Kontakt 80, das weitere Kontaktelement 76 und die Gelenklasche 36. Dieser Stromfluss ist in Figur 5 mit Minuszeichen angedeutet.

Das im Rohrschaft 12 angeordnete Kraftübertragungselement 30 in Form der Schub- und Zugstange ist zur Isolierung gegenüber dem Rohrschaft 12 von einer Umhüllung 84 aus nichtleitendem Material umgeben.

## Patentansprüche

1. Bipolares medizinisches Instrument, mit einem Schaft (12), mit zwei Maulteilen (16, 18), die relativ zueinander beweglich an einem distalen Ende (14) des Schaftes (12) angeordnet sind, wobei die Maulteile (16,18) jeweils eine mit Hochfrequenzstrom beaufschlagbare Elektrode (56, 58) unterschiedlicher Polarität bilden, wobei jedem Maulteil (16, 18) eine getrennte Stromzuleitung zugeordnet ist, von denen eine über den Schaft (12) und die andere durch ein in dem Schaft (12) angeordnetes, axial bewegliches Kraftübertragungselement (30), das kraftschlüssig mit zumindest einem Maulteil (16, 18) verbunden ist, gebildet wird, wobei die Maulteile (16, 18) elektrisch voneinander isoliert sind, und wobei ein distaler Endbereich des Kraftübertragungselementes (30) von einem kappenartigen oder hülsenartigen isolierenden Element (32) umschlossen ist, das aus einem elektrisch isolierenden Material ausgebildet und an dem Kraftübertragungselement (30) angebracht ist, und wobei das isolierende Element (32) gelenkig mit zumindest einem Maulteil (16, 18) verbunden ist, wobei ein elektrisch leitendes erstes Kontaktelement (76) vorhanden ist, mittels dessen der Stromfluss von dem Rohrschaft (12) auf das eine Maulteil (18) bewerkstelligbar ist, **dadurch gekennzeichnet, dass** das erste Kontaktelement (76) an dem isolierenden Element (32) fixiert ist.

2. Bipolares medizinisches Instrument nach Anspruch 1, **dadurch gekennzeichnet, dass** ein weiteres, einen Stromfluss von dem Übertragungselement (30) an das andere Maulteil (16) bewerkstelligendes zweites Kontaktelement (68) vorhanden ist, das in Bezug auf ein erstes Kontaktelement (76) umfänglich versetzt zu diesem angeordnet ist.

3. Bipolares medizinisches Instrument nach Anspruch 2, **dadurch gekennzeichnet, dass** das zweite Kontaktelement (68) über einen Kontakt (70) mit dem Kraftübertragungselement (30) verbunden ist, der als zumindest ein, durch einen Hülsenabschnitt (33) des isolierenden Elements (32) durchreichender Stift (72, 74) ausgebildet ist.

4. Bipolares medizinisches Instrument nach einem der Ansprüche 1 bis 3, **dadurch gekennzeichnet, dass** zumindest ein Maulteil (18) an der Seite, die dem anderen Maulteil (16) zugewandt ist, zumindest im Bereich einer Anlenkung der beiden Maulteile (16, 18) untereinander mit einer Isolierung (82) versehen ist.

5. Bipolares medizinisches Instrument nach einem der Ansprüche 1 bis 4, **dadurch gekennzeichnet, dass** ein Anlenkzapfen (38), über den die beiden Maulteile (16, 18) schwenkbar miteinander verbunden sind, von einem elektrisch isolierenden Material (39) umschlossen ist.

6. Bipolares medizinisches Instrument nach einem der Ansprüche 1 bis 5, **dadurch gekennzeichnet, dass** die elektrisch isolierenden Materialien einen Keramikwerkstoff, vorzugsweise einen Keramikwerkstoff hoher Härte und geringer Sprödigkeit, aufweisen.

7. Bipolares medizinisches Instrument nach Anspruch 1, **dadurch gekennzeichnet, dass** das isolierende Element (32) gelenkig mit beiden Maulteilen (16, 18) verbunden ist.

8. Bipolares medizinisches Instrument nach Anspruch 1, **dadurch gekennzeichnet, dass** das isolierende Element (32) einen Hülsenabschnitt (33) aufweist, der auf den distalen Endbereich des Kraftübertragungselementes (30) aufgebracht ist.

9. Bipolares medizinisches Instrument nach Anspruch 7, **dadurch gekennzeichnet, dass** das isolierende Element (32) einen T-Abschnitt (35) aufweist, an dem die gelenkige Verbindung mit dem/den Maulteilen (16, 18) bewerkstelligbar ist.

## Claims

1. Bipolar medical instrument having a shaft (12), further having two jaw parts (16, 18) which are arranged such that they can move relative to one another at a distal end (14) of the shaft (12), with the jaw parts (16, 18) each forming an electrode (56, 58), to which high frequency current can be applied, of different polarity, wherein each jaw part (16, 18) having an associated separate electrical power supply line, one of which is provided via the shaft (12) and the other is provided by an axially moving force transmission element (30) which is arranged in the shaft (12) and which is connected to at least one jaw part (16, 18) in a manner that force can be transmitted, wherein the jaw parts (16, 18) being electrically isolated from one another, and wherein a distal end area of the force transmission element (30) is surrounded by a cap-like or sleeve-like isolating element (32) which is made from an electrically insulating material and which is mounted at the force transmission element (30), and wherein the isolating element (32) is connected to at least one jaw part (16, 18) in an articulated manner, wherein an electrically conductive first contact member (76) is provided, by means of which the current flow can be provided from the tubular shaft to one jaw part (18), **characterized in that** the first contact element (76) is fixed to the isolating element (32).

2. Bipolar medical instrument of claim 1, **characterized in that** a further, second contact element (68), which provides a current flow from the transmission element (30) to the other jaw part (16), is provided and which is arranged circumferentially offset with respect to the first contact element (76).

3. Bipolar medical instrument of claim 2, **characterized in that** the second contact element (68) is connected via a contact (70) to the force transmission element (30), which is in the form of at least one pin (72, 74) which passes through a sleeve section (33) of the isolating element (32).

4. Bipolar medical instrument of anyone of claims 1 through 3, **characterized in that** at least one jaw part (18) is provided with an insulation (82) on the side which faces the other jaw part (16), at least in an area where the two jaw parts (16, 18) are articulated one to another.

5. Bipolar medical instrument of anyone of claims 1 through 4, **characterized in that** an articulation pivot (38), via which the two jaw parts (16, 18) are connected to one another such that they can pivot, is surrounded by an electrically insulating material (39).

6. Bipolar medical instrument of anyone of claims 1 through 5, **characterized in that** the electrically insulating materials have a ceramic material, preferably a ceramic material which is very hard and not very brittle.

7. Bipolar medical instrument of claim 1, **characterized in that** the isolating element (32) is connected to both jaw parts (16, 18) in an articulated manner.

8. Bipolar medical instrument of claim 1, **characterized in that** the isolating element (32) has a sleeve section (33) which is mounted on the distal end area of the force transmission element (30).

9. Bipolar medical instrument of claim 7, **characterized in that** the isolating element (32) has a T-section (35) on which the articulated connection either to the one jaw part or to the jaw parts (16, 18) can be provided.

## Revendications

1. Instrument médical bipolaire, comportant un corps (12) et deux mâchoires (16, 18) qui sont disposées à une extrémité distale (14) du corps (12) et mobiles l'une par rapport à l'autre, chacune des mâchoires (16, 18) formant une électrode (56, 58) de polarité différente pouvant être soumise à un courant à haute fréquence, une ligne d'alimentation électrique séparée étant associé à chaque mâchoire (16, 18), dont une est formée par le corps (12) et l'autre par un élément de transmission de force (30) mobile axialement, disposé dans le corps (12) et relié rigidement à au moins une mâchoire (16, 18), les mâchoires (16, 18) étant isolées électriquement l'une de l'autre et une partie terminale distale de l'élément de transmission de force (30) étant entourée par un élément isolant (32) en forme de capuchon ou de manchon qui est composé d'un matériau électriquement isolant et monté sur l'élément de transmission de force (30), et l'élément isolant (32) étant reliée de manière articulée à au moins une mâchoire (16, 18), un premier élément de contact (76) électriquement conducteur étant présent, qui permet de réaliser le flux de courant du corps tubulaire (12) vers une mâchoire (18), **caractérisé en ce que** le premier élément de contact (76) est fixé sur l'élément isolant (32).

2. Instrument médical bipolaire selon la revendication 1, **caractérisé en ce qu'**un autre ou deuxième élément de contact (68) réalisant un flux de courant de l'élément de transmission (30) vers l'autre mâchoire (16) est présent, lequel est disposé périphériquement à un premier élément de contact (76), en décalage par rapport à celui-ci.

3. Instrument médical bipolaire selon la revendication 2, **caractérisé en ce que** le deuxième élément de contact (68) est reliée par un contact (70) à l'élément de transmission de force (30) qui est réalisé sous la forme d'au moins une tige (72, 74) passant à travers une portion en forme de manchon (33) de l'élément isolant (32).

4. Instrument médical bipolaire selon une des revendications 1 à 3, **caractérisé en ce qu'**au moins une mâchoire (18) est pourvue d'une isolation (82) du côté tourné vers l'autre mâchoire (16), au moins dans la zone d'une articulation des deux mâchoires (16, 18).

5. Instrument médical bipolaire selon une des revendications 1 à 4, **caractérisé en ce qu'**un tourillon d'articulation (38), par lequel les deux mâchoires (16, 18) sont reliées ensemble de manière pivotante, est entouré par un matériau électriquement isolant (39).

6. Instrument médical bipolaire selon une des revendications 1 à 5, **caractérisé en ce que** les matériaux électriquement isolants présentent un matériau céramique, de préférence un matériau céramique à haute dureté et faible fragilité.

7. Instrument médical bipolaire selon la revendication 1, **caractérisé en ce que** l'élément isolant (32) est relié de manière articulée aux deux mâchoires (16, 18).

8. Instrument médical bipolaire selon la revendication 1, **caractérisé en ce que** l'élément isolant (32) présente une portion en forme de manchon (33) qui est montée sur la partie terminale distale de l'élément de transmission de force (30).

9. Instrument médical bipolaire selon la revendication 1, **caractérisé en ce que** l'élément isolant (32) présente une portion en T (35) sur laquelle la liaison articulée avec la/les mâchoire(s) (16, 18) est réalisable.
